(19) Europäisches Patentamt
European Patent Office
Office européen des brevets

(11) EP 1 261 424 B1

(12) **EUROPÄISCHE PATENTSCHRIFT**

(45) Veröffentlichungstag und Bekanntmachung des Hinweises auf die Patenterteilung:
**09.06.2004 Patentblatt 2004/24**

(21) Anmeldenummer: **01923638.9**

(22) Anmeldetag: **06.03.2001**

(51) Int Cl.[7]: **B01J 27/198**, C07C 51/215, C07C 51/25, C07C 57/145

(86) Internationale Anmeldenummer:
**PCT/EP2001/002492**

(87) Internationale Veröffentlichungsnummer:
**WO 2001/068245 (20.09.2001 Gazette 2001/38)**

(54) **HOHLZYLINDERFÖRMIGER KATALYSATOR UND VERFAHREN ZUR HERSTELLUNG VON MALEINSÄUREANHYDRID**

HOLLOW CYLINDRICAL CATALYST AND A METHOD FOR PRODUCING A MALEIC ACID ANHYDRIDE

CATALYSEUR EN FORME DE CYLINDRE CREUX ET PROCEDE DE FABRICATION D'HYDRURE D'ACIDE MALEIQUE

(84) Benannte Vertragsstaaten:
**AT BE CH CY DE DK ES FI FR GB GR IE IT LI LU MC NL PT SE TR**

(30) Priorität: **10.03.2000 DE 10011307**

(43) Veröffentlichungstag der Anmeldung:
**04.12.2002 Patentblatt 2002/49**

(73) Patentinhaber: **BASF AKTIENGESELLSCHAFT**
**67056 Ludwigshafen (DE)**

(72) Erfinder:
- **WEIGUNY, Jens**
  **67251 Freinsheim (DE)**
- **STORCK, Sebastian**
  **68165 Mannheim (DE)**
- **TENTEN, Andreas**
  **67487 Maikammer (DE)**

(56) Entgegenhaltungen:
**US-A- 4 283 307**
**US-A- 4 795 818**
**US-A- 5 168 090**

Bemerkungen:
Die Akte enthält technische Angaben, die nach dem Eingang der Anmeldung eingereicht wurden und die nicht in dieser Patentschrift enthalten sind.

EP 1 261 424 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die vorliegende Erfindung betrifft einen Katalysator für die Herstellung von Maleinsäureanhydrid durch heterogenkatalytische Gasphasenoxidation eines Kohlenwasserstoffs mit mindestens vier Kohlenstoffatomen, der eine katalytisch aktive Masse enthaltend Vanadium, Phosphor und Sauerstoff umfasst und eine im wesentlichen hohlzylinderförmige Struktur aufweist.

Ferner betrifft die vorliegende Erfindung ein Verfahren zur Herstellung von Maleinsäureanhydrid durch heterogenkatalytische Gasphasenoxidation eines Kohlenwasserstoffs mit mindestens vier Kohlenstoffatomen unter Verwendung des erfindungsgemäßen Katalysators.

Maleinsäureanhydrid ist ein wichtiges Zwischenprodukt bei der Synthese von γ-Butyrolacton, Tetrahydrofuran und 1,4-Butandiol, welche ihrerseits als Lösungsmittel eingesetzt werden oder beispielsweise zu Polymeren, wie Polytetrahydrofuran oder Polyvinylpyrrolidon weiterverarbeitet werden.

Zur Herstellung von Maleinsäureanhydrid durch heterogenkatalytische Gasphasenoxidation von Kohlenwasserstoffen werden im allgemeinen tablettenförmige Katalysatoren, d.h. sogenannte Vollzylinder, eingesetzt, welche Phosphor, Vanadium und Sauerstoff enthaltenden. Derartige Katalysatoren sind beispielsweise beschrieben in den US-Patenten 5,275,996 oder 5,641,722.

In T.P. Wellauer et al., Chem. Eng. Sci., Vol. 41, No. 4, 1986, Seite 765 bis 772 wurde bei der Untersuchung der Partialoxidation von n-Butan zu Maleinsäureanhydrid an Phosphor, Vanadium und Sauerstoff enthaltenden Katalysatoren erkannt, daß gegenüber den üblicherweise industriell eingesetzten 3 mm × 3 mm Tabletten (Durchmesser × Höhe) sogenannte Ringe mit der Geometrie 8 mm × 5 mm × 5 mm (äußerer Durchmesser × Höhe × Durchmesser des inneren Lochs) einen etwa um 65% höheren Wärmeaustrag aus dem Katalysatorbett, bezogen auf eine Volumeneinheit, besitzen. Nach den beschriebenen Simulationen kann auch die Ausbeute an Maleinsäureanhydrid durch Einsatz der 5 mm × 8 mm × 5 mm Ringe ("Hohlzylinder") gegenüber 2,5 mm × 2,5 mm Tabletten ("Vollzylinder") um 5 bis 7% erhöht werden.

In einer Reihe von Veröffentlichungen zur Herstellung von Maleinsäureanhydrid durch heterogenkatalytische Gasphasenoxidation von Kohlenwasserstoffen ist der Einsatz von Phosphor, Vanadium und Sauerstoff enthaltenden Katalysatoren mit ringförmiger (hohlzylinderförmiger) Struktur vorbeschrieben. So sind in US 4,713,464 Ringe der Geometrie 5 mm × 4 mm × 2 mm, in EP-A 0 593 646 Ringe der Geometrie 8 mm × 8 mm × 4 mm, in US 4,795,818 Ringe der Geometrie 6,35 mm × 3,18 mm × 3,18 mm und in US 5,296,436 Ringe der Geometrie 4,763 mm × 4,763 × 1,588 mm offenbart.

Im US-Patent 4,283,307 wird eine Vanadium, Phosphor und Sauerstoff enthaltende Katalysatorstruktur in Form eines Hohlzylinders beschrieben, deren äußerer Durchmesser 3,969 bis 4,762 mm, deren Höhe 3,969 bis 4,762 mm und deren innerer Durchmesser 0,888 bis 7,925 mm beträgt. Der Durchmesser des inneren Lochs des Hohlzylinders beträgt üblicherweise 30 bis 50% des äußeren Durchmessers, wobei Höhe und äußerer Durchmesser bevorzugt gleich sind. In den Beispielen ist eine Hohlzylinderstruktur mit der Geometrie 3,969 mm × 3,969 mm × 1,587 mm offenbart. Gegenüber 3,969 mm × 3,969 mm Tabletten mit identischer Aktivkomponente wurde bei Einsatz der Hohlzylinder eine Ausbeutesteigerung an Maleinsäureanhydrid von bis zu 24 rel.-% erhalten.

Das US-Patent 5,168,090 offenbart eine Katalysatorstruktur für die Herstellung von Maleinsäureanhydrid durch heterogenkatalytische Gasphasenoxidation von Kohlenwasserstoffen, welche mindestens einen geordneten Hohlraum in der äußeren Oberfläche umfaßt, ein geometrisches Volumen $V_{geo}$ von 30 bis 67% von dem theoretischen Volumen $V_{overall}$, das die hohlraumfreie, massive Struktur mit gleichem Außendurchmesser und gleicher Höhe aufweisen würde und ein Verhältnis der geometrischen Oberfläche $A_{geo}$ zum geometrischen Volumen $V_{geo}$ von mindestens 20 $cm^{-1}$ aufweist. Die als Vergleichsbeispiel genannten Hohlzylinder besitzen eine Höhe von 4,76, 4,29 sowie 4,14 mm, einen äußeren Durchmesser von jeweils 4,76 mm und einen inneren Durchmesser von jeweils 1,58 mm.

Die Aufgabe der vorliegenden Erfindung bestand darin, einen Katalysator zur Herstellung von Maleinsäureanhydrid durch heterogenkatalytische Gasphasenoxidation von Kohlenwasserstoffen zu finden, welcher leicht herstellbar ist, einen geringen Druckverlust bei ausreichend hoher mechanischer Stabilität besitzt und gegenüber den Katalysatoren nach dem Stand der Technik eine hohe Kohlenwasserstoff-Belastung des Katalysators ermöglicht und dabei einen hohen Umsatz, eine hohe Selektivität, eine hohe Ausbeute und daher eine hohe Raum/Zeit-Ausbeute ermöglicht.

Demgemäß wurde ein Katalysator für die Herstellung von Maleinsäureanhydrid durch heterogenkatalytische Gasphasenoxidation eines Kohlenwasserstoffs mit mindestens vier Kohlenstoffatomen, der eine katalytisch aktive Masse enthaltend Vanadium, Phosphor und Sauerstoff umfasst und eine im wesentlichen hohlzylinderförmige Struktur aufweist, gefunden, der dadurch gekennzeichnet ist, daß die hohlzylinderförmige Struktur (a) ein Verhältnis der Höhe h zum Durchmesser der hindurchgehenden Öffnung $d_2$ von höchstens 1,5 und (b) ein Verhältnis der geometrischen Oberfläche $A_{geo}$ zum geometrischen Volumen $V_{geo}$ von mindestens 2 $mm^{-1}$ aufweist.

Unter einer im wesentlichen hohlzylinderförmigen Struktur ist eine Struktur zu verstehen, welche im wesentlichen einen Zylinder mit einer zwischen den beiden Deckelflächen hindurchgehenden Öffnung umfaßt. Der Zylinder ist charakterisiert durch zwei im wesentlichen parallele Deckelflächen und einer Mantelfläche, wobei der Querschnitt

des Zylinders, d.h. parallel zu den Deckelflächen, im wesentlichen von kreisförmiger Struktur ist. Der Querschnitt der hindurchgehenden Öffnung, d.h. parallel zu den Dekkelflächen des Zylinders, ist im wesentlichen ebenfalls von kreisförmiger Struktur. Bevorzugt befindet sich die hindurchgehende Öffnung mittig zu den Deckelflächen, wobei andere räumliche Anordnungen damit nicht ausgeschlossen sind.

Der Begriff "im wesentlichen" weißt darauf hin, daß Abweichungen von der Idealgeometrie, wie beispielsweise leichte Deformationen der kreisförmigen Struktur, nicht planparallel ausgerichtete Dekkelflächen, abgeplatzte Ecken und Kanten, Oberflächenrauhigkeit oder Einkerbungen in der Mantelfläche, den Deckelflächen oder der Innenfläche der hindurchgehenden Bohrung beim erfindungsgemäßen Katalysator mit umfaßt sind. Im Rahmen der Genauigkeit der Tablettierkunst sind kreisförmige Deckelflächen, ein kreisförmiger Querschnitt der hindurchgehenden Bohrung, parallel ausgerichtete Deckelflächen und makroskopisch glatte Oberflächen bevorzugt.

Die im wesentlichen hohlzylinderförmige Struktur kann beschrieben werden durch einen äußeren Durchmesser $d_1$, einer Höhe h als Abstand der beiden Deckelflächen und einem Durchmesser des inneren Lochs (hindurchgehende Öffnung) $d_2$. Bei den drei genannten Größen sind jeweils die gemittelten Werte des Hohlzylinders zu verstehen. Dies gilt insbesondere bei Abweichungen von der Idealgeometrie.

Der erfindungsgemäße Katalysator weist ein Verhältnis von der Höhe h zum Durchmesser der hindurchgehenden Öffnung $d_2$ von höchstens 1,5 auf. Bevorzugt beträgt der Quotient $h/d_2$ 0,5 bis 1,5, besonders bevorzugt 1,0 bis 1,5.

Als weitere charakteristische Eigenschaft weist der Katalysator ein Verhältnis der geometrischen Oberfläche $A_{geo}$ zum geometrischen Volumen $V_{geo}$ von mindestens 2 $mm^{-1}$ auf. Unter der geometrischen Oberfläche $A_{geo}$ ist die rechnerische Oberfläche aller Außenflächen des Hohlzylinders, inklusive der inneren Mantelfläche der hindurchgehenden Öffnung unter Zugrundelegung der oben genannten Größen $d_1$, h und $d_2$ zu verstehen. Unter dem geometrischen Volumen $V_{geo}$ ist das rechnerische Volumen des Hohlzylinders unter Zugrundelegung der oben genannten Größen $d_1$, h und $d_2$ zu verstehen. Bei der Berechnung beider Größen bleiben somit Poren ebenso unberücksichtigt wie etwa Einkerbungen oder Rauhigkeiten der Außenflächen. Bevorzugt beträgt der Quotient $A_{geo}/V_{geo}$ 2 bis 3 $mm^{-1}$, besonders bevorzugt 2 bis 2,5 $mm^{-1}$.

In einer bevorzugten Ausführungsform ist der erfindungsgemäße Katalysator zusätzlich charakterisiert durch das Verhältnis des geometrischen Volumens $V_{geo}$ der hohlzylinderförmigen Struktur zum theoretischen Volumen $V_{overall}$ eines entsprechenden Vollzylinders mit gleicher Höhe h und gleichem äußeren Durchmesser $d_1$, welches höchstens 0,85 beträgt. Das theoretische Volumen $V_{overall}$ eines entsprechenden Vollzylinders mit gleicher Höhe h und gleichem äußeren Durchmesser $d_1$ ist dabei ebenfalls rechnerisch unter Zugrundelegung der oben genannten Größen $d_1$ und h zu ermitteln. Besonders bevorzugt beträgt der Quotient $V_{geo}/V_{overall}$ 0,3 bis 0,85, ganz besonders bevorzugt 0,6 bis 0,85, insbesondere 0,7 bis 0,85.

Der äußere Durchmesser $d_1$ des erfindungsgemäßen Katalysators beträgt bevorzugt 3 bis 10 mm, besonders bevorzugt 4 bis 8 mm, ganz besonders bevorzugt 5 bis 6 mm. Die Höhe h beträgt bevorzugt 1 bis 10 mm, besonders bevorzugt 2 bis 6 mm, ganz besonders bevorzugt 2 bis 3 mm. Der Durchmesser der hindurchgehenden Öffnung $d_2$ beträgt bevorzugt 1 bis 8 mm, besonders bevorzugt 2 bis 6 mm, ganz besonders bevorzugt 2 bis 3 mm.

Die erfindungsgemäßen Katalysatoren umfassen als katalytisch aktive Masse eine sauerstoffhaltige Vanadium-Phosphor-Verbindung oder Gemische solcher Verbindungen. Geeignete Aktivmassen sind beispielsweise in den Patentschriften US 5,275,996, US 5,641,722, US 5,137,860, US 5,095,125 oder US 4,933,312 beschrieben.

Die erfindungsgemäßen Katalysatoren können des weiteren sogenannte Promotoren enthalten. Als geeignete Promotoren sind die Elemente der 1. bis 15. Gruppe des Periodensystems sowie deren Verbindungen genannt. Geeignete Promotoren sind beispielsweise in den Offenlegungsschriften WO 97/12674 und WO 95/26817 sowie in den Patenten US 5,137,860, US 5,296,436, US 5,158,923 und US 4,795,818 beschrieben. Bevorzugt werden als Promotoren Verbindungen der Elemente Kobalt, Molybdän, Eisen, Zink, Hafnium, Zirkon, Lithium, Titan, Chrom, Mangan, Nickel, Kupfer, Bor, Silicium, Antimon, Zinn, Niob und Wismut, besonders bevorzugt Molybdän, Eisen, Zink, Antimon, Wismut, Lithium. Die promotierten erfindungsgemäßen Katalysatoren können einen oder mehrere Promotoren enthalten. Der Gehalt an Promotoren beträgt in Summe im fertigen Katalysator im allgemeinen nicht mehr als etwa 5 Gew.-%, jeweils als Oxid gerechnet.

Die erfindungsgemäßen Katalysatoren können auch sogenannte Hilfsmittel, wie etwa Tablettierhilfsmittel oder Porenbildner enthalten.

Tablettierhilfsmittel werden im allgemeinen zugesetzt, wenn die Formgebung der erfindungsgemäßen Katalysatoren über eine Tablettierung erfolgt. Tablettierhilfsmittel sind in der Regel katalytisch inert und verbessern die Tablettiereigenschaften des sogenannten Precursorpulvers, einer Zwischenstufe in der Katalysatorherstellung, beispielsweise durch Erhöhung der Gleit- und Rieselfähigkeit. Als geeignetes und bevorzugtes Tablettierhilfsmittel sei Graphit genannt. Die zugesetzten Tablettierhilfsmittel verbleiben in der Regel im aktivierten Katalysator. Typischerweise liegt der Gehalt an Tablettierhilfsmittel im fertigen Katalysator bei etwa 2 bis 6 Gew.-%.

Porenbildner sind Stoffe, welche zur gezielten Einstellung der Porenstruktur im Makroporenbereich eingesetzt werden. Sie können prinzipiell unabhängig vom Formgebungsverfahren eingesetzt werden. In der Regel handelt es

sich um Kohlenstoff, Wasserstoff, Sauerstoff und/oder Stickstoff enthaltende Verbindungen, welche vor der Formgebung des Katalysator zugesetzt werden und bei der anschließenden Aktivierung des Katalysators unter Sublimation, Zersetzung und/oder Verdampfung zum überwiegenden Teil wieder entfernt werden. Der fertige Katalysator kann dennoch Rückstände oder Zersetzungsprodukte des Porenbildners enthalten.

Die erfindungsgemäßen Katalysatoren können die Vanadium, Phosphor und Sauerstoff enthaltende Aktivmasse beispielsweise in reiner, unverdünnter Form als sogenannter "Vollkatalysator" oder verdünnt mit einem bevorzugt oxidischen Trägermaterial als sogenannter "Mischkatalysator" enthalten. Als geeignete Trägermaterialien für die Mischkatalysatoren seien beispielsweise Aluminiumoxid, Siliciumdioxid, Alumosilikate, Zirkondioxid, Titandioxid oder Gemische davon genannt. Bevorzugt sind die Voll- und Mischkatalysatoren, besonders bevorzugt die Vollkatalysatoren.

Die erfindungsgemäßen Katalysatoren besitzen bevorzugt ein Phosphor/Vanadium-Atomverhältnis von 0,9 bis 1,5, besonders bevorzugt von 0,9 bis 1,2 und ganz besonders bevorzugt von 1,0 bis 1,1. Die mittlere Oxidationsstufe des Vanadiums beträgt bevorzugt +3,9 bis +4,4 und besonders bevorzugt 4,0 bis 4,3. Die erfindungsgemäßen Katalysatoren besitzen bevorzugt eine BET-Oberfläche von 10 bis 50 $m^2/g$ und besonders bevorzugt von 15 bis 30 $m^2/g$. Sie weisen bevorzugt ein Porenvolumen von 0,1 bis 0,5 ml/g und besonders bevorzugt von 0,1 bis 0,3 ml/g auf. Die Schüttdichte der erfindungsgemäßen Katalysatoren beträgt bevorzugt 0,5 bis 1,5 kg/l und besonders bevorzugt 0,5 bis 1,0 kg/l.

Die erfindungsgemäßen Katalysatoren können beispielsweise wie in den Patentschriften US 5,275,996 und US 5,641,722 oder der Offenlegungsschrift WO 97/12674 beschrieben hergestellt werden, wobei selbstverständlich die Formgebung in die erfindungsgemäße hohlzylinderförmige Struktur erfolgt. Die Formgebung erfolgt bevorzugt durch Tablettierung.

Die wesentlichen Schritte der bevorzugten Katalysatorherstellung unter Bildung eines sogenannten Precursorpulvers, Formgebung und anschließende Aktivierung sind im folgenden erläutert.

a) Umsetzung einer fünfwertigen Vanadiumverbindung (z.B. $V_2O_5$) mit einem organischen, reduzierenden Lösungsmittel (z.B. Alkohol, wie etwa Isobutanol) in Gegenwart einer fünfwertigen Phosphorverbindung (z.B. Ortho- und/oder Pyrophosphorsäure) unter Erwärmen. Gegebenenfalls kann diese Stufe in Gegenwart eines dispergierten, pulverförmigen Trägermaterials durchgeführt werden. Bevorzugt ist die Umsetzung ohne Zusatz von Trägermaterial.

b) Isolierung des gebildeten Vanadium-, Phosphor-, Sauerstoff enthaltenden Katalysatorprecursors ("VPO-Precursor"), z.B. durch Filtration oder Eindampfung.

c) Trocknung des VPO-Precursors, gegebenenfalls beginnende Präformierung durch zusätzliche Wasserabspaltung aus dem VPO-Precursor. Dem getrockneten VPO-Precursor-Pulver kann nun gegebenenfalls pulverförmiges Trägermaterial und/oder ein sogenannter Porenbildner, wie beispielsweise Stearinsäure, Cellulose oder Paraffine untermischt werden. Bevorzugt ist die Weiterverarbeitung ohne Zusatz eines Trägermaterials.

d) Formgebung durch Überführung in die erfindungsgemäße, im wesentlichen hohlzylinderförmige Struktur. Die Formgebung erfolgt bevorzugt durch Tablettierung, vorteilhafterweise unter vorheriger Untermischung eines sogenannten Gleitmittels, wie etwa Graphit.

e) Präformierung des geformten VPO-Precursors durch Erhitzen in einer Atmosphäre, welche Sauerstoff, Stickstoff, Edelgase, Kohlendioxid, Kohlenmonoxid und/oder Wasserdampf enthält. Durch geeignete, an das jeweilige Katalysatorsystem angepaßte Kombination von Temperaturen, Behandlungsdauern und Gasatmosphären kann die mechanische und katalytische Eigenschaft des Katalysators beeinflußt werden.

Als weniger bevorzugte Alternative zur Tablettierung sei beispielsweise die Extrusion genannt. Bei dieser Variante teigt man beispielsweise den in (b) erhaltenen VPO-Precursor an, um eine extrusionsfähige Masse zu erhalten. Diese kann dann in die erfindungsgemäße hohlzylinderförmige Struktur extrutiert werden. Nach der Trocknung kann nun die Präformierung analog (e) erfolgen.

Es ist auch möglich, zuerst das Pulver wie unter (a) bis (c) und (e) beschrieben zu behandeln und erst anschließend das präformierte Pulver anzuteigen und zu extrudieren. Nach der Extrusion werden die Formkörper getrocknet beziehungsweise erneut getempert.

In einer besonders bevorzugten Ausführungsform zur Herstellung des Katalysators gibt man Vanadiumpentoxidpulver $V_2O_5$ in Isobutanol und versetzt die Mischung mit der für die Einstellung des gewünschten Phosphor/ Vanadium-Atomverhältnisses erforderlichen Menge an Phosphorsäure. Anschließend erwärmt man die Mischung, wobei sich unter Reduktion des Vanadiums und der Umsetzung mit der Phosphorsäure der VPO-Katalysator-Precursor

bildet. Dieser wird beispielsweise durch Filtration isoliert, gegebenenfalls gewaschen und bei einer Temperatur über 100°C getrocknet und gegebenenfalls vorformiert. Das erhaltene Precursor-Pulver wird nun mit einem Gleitmittel, bevorzugt Graphit, und gegebenenfalls einem Porenbildner, beispielsweise Stearinsäure, vermischt und durch Tablettierung in die hohlzylinderförmige Struktur mit den erfindungsgemäßen geometrischen Bedingungen überführt. Die Formkörper werden nun durch Erhitzen in einer Atmosphäre, enthaltend Sauerstoff, Stickstoff, Edelgase, Kohlendioxid, Kohlenmonoxid und/oder Wasserdampf präformiert.

Die erfindungsgemäßen Katalysatoren zeichnen sich durch ihre besondere hohlzylinderförmige Struktur und die besonderen geometrischen Bedingungen aus. Sie sind aus an sich bekannten Aktivmassen leicht herstellbar und besitzen bei ihrem Einsatz in der heterogenkatalytischen Gasphasenoxidation einen geringen Druckverlust bei ausreichend hoher mechanischer Stabilität. Sie besitzen des weiteren, bezogen auf ihr geometrisches Volumen, eine große geometrische Oberfläche, was zu einem entscheidenden Vorteile in der Aktivität und Selektivität führt. Durch die hindurchgehende Öffnung wird des weiteren Aktivmasse eingespart und das Schüttgewicht verringert.

Gegenüber den vorbeschriebenen Geometrien besitzen die erfindungsgemäßen Katalysatoren entscheidende Vorteile, insbesondere gegenüber

| | |
|---|---|
| Tabletten (Vollzylinder) | - weniger Aktivmasse<br>- geringeres Schüttgewicht<br>- höhere Aktivität<br>- höhere Selektivität<br>- geringerer Druckverlust |
| Hohlzylinder vorbeschriebener Geometrien | - höhere Aktivität<br>- höhere Selektivität |
| Trilobes, Tristars | - einfachere Herstellung<br>- höhere Stabilität. |

Weiterhin ist Gegenstand der Erfindung ein Verfahren zur Herstellung von Maleinsäureanhydrid durch heterogenkatalytische Gasphasenoxidation eines Kohlenwasserstoffs mit mindestens vier Kohlenstoffatomen mit Sauerstoff enthaltenden Gasen unter Einsatz des erfindungsgemäßen Katalysators.

Als Reaktoren werden im allgemeinen Rohrbündelreaktoren eingesetzt. Ein Rohrbündelreaktor besteht wiederum aus mindestens einem Reaktorrohr, welches zur Beheizung und/oder Kühlung von einem Wärmeträgermedium umgeben ist. Im allgemeinen enthalten die technisch eingesetzten Rohrbündelreaktoren wenige hundert bis mehrere zehntausend parallel-geschaltete Reaktorrohre.

Die Rohrbündelreaktoren können eine oder mehrere Vorheizzonen enthalten, welche das eintretende Gasgemisch aufheizen. Eine in einem Rohrbündelreaktor integrierte vorheizzone kann beispielsweise durch mit Inertmaterial gefüllte Reaktorrohre, welche ebenfalls von Wärmeträgermedium umgeben sind, realisiert werden. Als Inertmaterial sind prinzipiell alle Formkörper geeignet, welche chemisch inert sind, d.h. keine heterogenkatalytische Reaktion induzieren oder katalysieren, und welche einen maximalen Druckverlust unterhalb des jeweiligen, maximal tolerierbaren, anlagenspezifischen Wert aufweisen. Geeignet sind beispielsweise oxidische Materialen, wie etwa $Al_2O_3$, SiC oder metallische Materialien, wie etwa Edelstahl. Als Formkörper seien beispielsweise Kugeln, Tabletten, Hohlzylinder, Ringe, Trilobes, Tristars, Wagenräder, Extrudate oder regellos gebrochene Formkörper genannt.

Als Kohlenwasserstoffe sind im erfindungsgemäßen Verfahren aliphatische und aromatische, gesättigte und ungesättigte Kohlenwasserstoffe mit mindestens vier Kohlenstoffatomen, beispielsweise 1,3-Butadien, 1-Buten, 2-cis-Buten, 2-trans-Buten, n-Butan, $C_4$-Gemisch, 1,3-Pentadien, 1,4-Pentadien, 1-Penten, 2-cis-Penten, 2-trans-Penten, n-Pentan, Cyclopentadien, Dicyclopentadien, Cyclopenten, Cyclopentan, $C_5$-Gemisch, Hexene, Hexane, Cyclohexan und Benzol. Bevorzugt eingesetzt werden 1-Buten, 2-cis-Buten, 2-trans-Buten, n-Butan, Benzol oder deren Mischungen geeignet. Besonders bevorzugt ist der Einsatz von n-Butan und n-Butan-haltigen Gasen und Flüssigkeiten. Das verwendete n-Butan kann beispielsweise aus dem Erdgas, aus Steamcrackern oder FCC-Crackern stammen.

Die Zugabe des Kohlenwasserstoffs erfolgt im allgemeinen mengengeregelt, d.h. unter stetiger Vorgabe einer definierten Menge pro Zeiteinheit. Der Kohlenwasserstoff kann in flüssiger oder gasförmiger Form dosiert werden. Bevorzugt ist die Dosierung in flüssiger Form mit anschließender Verdampfung vor Eintritt in den Rohrbündelreaktor.

Als Oxidationsmittel werden Sauerstoff enthaltende Gase, wie beispielsweise Luft, synthetische Luft, ein mit Sauerstoff angereichertes Gas oder auch sogenannter "reiner", d.h. z.B. aus der Luftzerlegung stammender Sauerstoff eingesetzt. Auch das Sauerstoff-enthaltende Gas wird mengengeregelt zugegeben.

Das durch den Rohrbündelreaktor zu leitende Gas enthält im allgemeinen Inertgas. Üblicherweise beträgt der Inertgasanteil zu Beginn 50 bis 95 Vol.-%. Inertgase sind alle Gase, welche nicht direkt zu einer Bildung an Maleinsäureanhydrid beitragen, wie beispielsweise Stickstoff, Edelgase, Kohlenmonoxid, Kohlendioxid, Wasserdampf,

oxygenierte und nicht-oxygenierte Kohlenwasserstoffe mit weniger als vier Kohlenstoffatomen (z.B. Methan, Ethan, Propan, Methanol, Formaldehyd, Ameisensäure, Ethanol, Acetyaldehyd, Essigsäure, Propanol, Propionaldehyd, Propionsäure, Acrolein, Crotonaldehyd) und deren Mischungen. Im allgemeinen wird das Inertgas über das Sauerstoffenthaltende Gas in das System eingebracht. Es ist aber auch möglich, weitere Inertgase separat zuzuführen. Eine Anreicherung mit weiteren Inertgasen, welche beispielsweise aus der Partialoxidation der Kohlenwasserstoffe stammen können, ist über eine partielle Rückführung des gegebenenfalls aufbereiteten Reaktionsaustrags möglich.

Zur Gewährung einer langen Katalysatorstandzeit und weiteren Erhöhung von Umsatz, Selektivität, Ausbeute, Katalysator-Belastung und Raum/Zeit-Ausbeute wird dem Gas beim erfindungsgemäßen Verfahren bevorzugt eine flüchtige Phosphorverbindung zugeführt. Ihre Konzentration beträgt zu Beginn, d.h. am Reaktoreingang, mindestens 0,2 Volumen-ppm, d.h. $0{,}2 \cdot 10^{-6}$ Volumenanteile der flüchtigen Phosphorverbindungen bezogen auf das Gesamtvolumen des Gases am Reaktoreingang. Bevorzugt ist ein Gehalt von 0,2 bis 20 Volumen-ppm, besonders bevorzugt von 0,5 bis 10 Volumen-ppm. Als flüchtige Phosphorverbindungen sind all jene Phosphor-enthaltende Verbindungen zu verstehen, welche in der gewünschten Konzentration unter den Einsatzbedingungen gasförmig vorliegen. Beispielsweise seien die allgemeinen Formeln (I) und (II) genannt

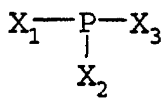 (I) und 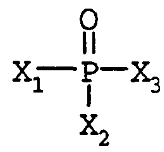 (II)

wobei $X^1$, $X^2$ und $X^3$ unabhängig voneinander Wasserstoff, Halogen, $C_1$- bis $C_6$-Alkyl, $C_3$-bis $C_6$-Cycloalkyl, $C_6$- bis $C_{10}$-Aryl, $C_1$- bis $C_6$-Alkoxy, $C_3$-bis $C_6$-Cycloalkoxy und $C_6$- bis $C_{10}$-Aroxy bedeuten. Bevorzugt sind Verbindungen der Formel (III)

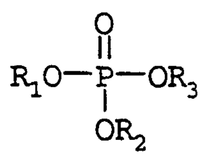 (III)

wobei $R^1$, $R^2$ und $R^3$ unabhängig voneinander Wasserstoff, $C_1$- bis $C_6$-Alkyl, $C_3$-bis $C_6$-Cycloalkyl und $C_6$- bis $C_{10}$-Aryl bedeuten. Besonders bevorzugt sind die Verbindungen der Formel (III), bei denen $R^1$, $R^2$ und $R^3$ unabhängig voneinander $C_1$- bis $C_4$-Alkyl bedeuten, beispielsweise Methyl, Ethyl, Propyl, 1-Methylethyl, Butyl, 1-Methylpropyl, 2-Methylpropyl und 1,1-Dimethylethyl. Ganz besonders bevorzugt sind Trimethylphosphat, Triethylphosphat und Tripropylphosphat, insbesondere Triethylphosphat.

Das erfindungsgemäße Verfahren wird im allgemeinen bei einer Temperatur von 350 bis 480°C durchgeführt. Unter der genannten Temperatur wird die Temperatur der im Rorbündelreaktor befindlichen Katalysatorschüttung verstanden, welche bei Ausübung des Verfahrens in Abwesenheit einer chemischen Reaktion vorliegen würde. Ist diese Temperatur nicht an allen Stellen exakt gleich, so meint der Begriff den Zahlenmittelwert der Temperaturen längs der Reaktionszone. Insbesondere bedeutet dies, daß die wahre, am Katalysator vorliegende Temperatur aufgrund der Exothermie der Oxidationsreaktion auch außerhalb des genannten Bereichs liegen kann. Bevorzugt wird das erfindungsgemäße Verfahren bei einer Temperatur von 380 bis 460°C, besonders bevorzugt 380 bis 430°C durchgeführt.

Das erfindungsgemäße Verfahren kann bei einem Druck unterhalb von Normaldruck (z.B. bis 0,05 MPa abs) als auch oberhalb von Normaldruck (z.B. bis 10 MPa abs) ausgeübt werden. Darunter ist der in der Rohrbündelreaktor-Einheit vorliegende Druck zu verstehen. Bevorzugt ist ein Druck von 0,1 bis 1 MPa abs, besonders bevorzugt 0,1 bis 0,5 MPa abs.

Bezüglich des Einsatzes des erfindungsgemäßen Katalystors im erfindungsgemäßen verfahren sind verschiedene Varianten möglich. Im einfachsten Fall wird der Rohrbündelreaktor mit der gleichen Katalysatorschüttung befüllt. Unter Katalysatorschüttung ist Katalysatormaterial zu verstehen, welches pro Volumeneinheit im Mittel die gleiche Zusammensetzung und die gleiche Aktivität besitzt. Eine Katalysatorschüttung kann sich zusammensetzen aus dem erfindungsgemäßen Katalysator, einer Mischung aus mindestens einem erfindungsgemäßen Katalysator und weiteren erfindungsgemäßen und/oder nicht-erfindungsgemäßen Katalysatoren, wobei die Katalysatorschüttung auch mit einem Inertmaterial durchmischt, d.h. "verdünnt" sein kann. In einer anderen Variante werden zwei oder mehr als zwei aufeinanderfolgende Katalysatorschüttungen im Rohrbündelreaktor eingesetzt. Es ist somit beispielsweise möglich und gegebenenfalls vorteilhaft, in der Nähe des Reaktoreingangs eine weniger aktive Katalysatorschüttung einzusetzen und in Durchleitungsrichtung dahinter, eine aktivere Katalysatorschüttung zu verwenden.

Das erfindungsgemäßen Verfahren kann in zwei bevorzugten Verfahrensvarianten, der Variante mit "geradem Durchgang" und der Variante mit "Rückführung" durchgeführt werden.

a) "gerader Durchgang"

Die Verfahrensvariante des "geraden Durchgangs" ist dadurch gekennzeichnet, daß der Umsatz an Kohlenwasserstoffen pro Reaktordurchgang 75 bis 95% beträgt und man aus dem Reaktoraustrag Maleinsäureanhydrid und gegebenenfalls oxygenierte Kohlenwasserstoff-Nebenprodukte entfernt, wobei das durch den Reaktor geleitete Reaktionsgas, insbesondere die nicht-umgesetzten Kohlenwasserstoffe, keiner direkten Rückführung zugeführt wird/werden. Bevorzugt beträgt der Gesamtumsatz an Kohlenwasserstoffen pro Reaktordurchgang 80 bis 90%. Der verbleibende Reststrom, welcher Inertgase, nicht-umgesetzte Kohlenwasserstoffe sowie gegebenenfalls weitere, nicht-abgetrennte Komponenten enthält, wird im allgemeinen aus der Anlage ausgeschleust.

Die Konzentration an Kohlenwasserstoffen beträgt zu Beginn, d.h. am Reaktoreingang, bevorzugt 1,0 bis 4,0 vol.-%, besonders bevorzugt 1,5 bis 3,0 vol.-%. Die Konzentration an Sauerstoff beträgt zu Beginn bevorzugt 5 bis 50 vol.-%, besonders bevorzugt 15 bis 30 vol.-%. Die Herkunft des eingesetzten Sauerstoffs ist für das erfindungsgemäße Verfahren prinzipiell unbedeutend, sofern keine schädlichen Verunreinigungen zugegen sind. Aus einfachen technischen Erwägungen ist als Sauerstoffquelle Luft bevorzugt. Diese kann im einfachsten Fall direkt oder bevorzugt nach einer Partikelreinigung eingesetzt werden. Eine Anreicherung von Sauerstoff, beispielsweise durch Luftverflüssigung und anschließender Destillation oder Druckwechseladsorption, ist prinzipiell möglich.

Die Belastung des Katalysators mit Kohlenwasserstoffen beträgt im allgemeinen mindstens 20 Nl/l·h, bevorzugt mindestens 30 Nl/l·h, besonders bevorzugt mindestens 35 Nl/l·h.

Die Abtrennung von Maleinsäureanhydrid kann beispielsweise durch Absorption in einem geeigneten Absorptionsmittel erfolgen. Geeignete Absorptionsmittel sind beispielsweise Wasser oder organische Flüssigkeiten. Bei Absorption in Wasser wird Maleinsäureanhydrid zu Maleinsäure hydratisiert. Bevorzugt ist die Absorption in einem organischen Lösungsmittel. Geeignete organische Lösungsmittel sind beispielsweise die in WO 97/43242 genannten hochsiedenden Lösungsmittel, wie Trikresylphosphat, Dibutylmaleat, hochmolekulares Wachs, aromatische Kohlenwasserstoffe mit einem Siedepunkt über 140°C oder Di-$C_4$-$C_8$-alkylphthalate, wie etwa Dibutylphthalat. In den genannten Lösungsmitteln werden im allgemeinen auch oxygenierte Kohlenwasserstoff-Nebenprodukte absorbiert. Die Absorption kann beispielsweise bei einer Temperatur von 60 bis 160°C und einem Druck von 0,1 bis 0,5 MPa abs oder darüber durchgeführt werden. Geeignete Verfahrensweisen sind etwa die Durchleitung des gasförmigen, gegebenenfalls abgekühlten Reaktoraustrags durch einen mit Absorptionsflüssigkeit gefüllten Behälter oder das Versprühen der Absorptionsflüssigkeit im Gasstrom. Entsprechende Methoden zum Auswaschen von Gasströmen sind dem Fachmann bekannt.

b) "Rückführung"

Die Verfahrensvariante der "Rückführung" ist dadurch gekennzeichnet, daß der Umsatz an Kohlenwasserstoffen pro Reaktordurchgang 30 bis 60% beträgt, man aus dem Reaktoraustrag Maleinsäureanhydrid und gegebenenfalls oxygenierte Kohlenwasserstoff-Nebenprodukte entfernt und mindestens einen Teil des verbleibenden Stromes oder wenigstens einen Teil der nicht-umgesetzten, gegebenenfalls abgetrennten Kohlenwasserstoffe in die Reaktionszone rückführt. Bevorzugt beträgt der Gesamtumsatz an Kohlenwasserstoffen pro Reaktordurchgang 40 bis 50%.

Die Konzentration an Kohlenwasserstoffen beträgt zu Beginn, d.h. am Reaktoreingang, bevorzugt mindestens 2,0 vol.-%, besonders bevorzugt mindestens 2,5 vol.-%. Die Konzentration an Sauerstoff beträgt zu Beginn bevorzugt 5 bis 60 vol.-%, besonders bevorzugt 15 bis 50 vol.-%. Die Herkunft des eingesetzten Sauerstoffs ist für das erfindungsgemäße Verfahren prinzipiell unbedeutend, sofern keine schädlichen Verunreinigungen zugegen sind. Aus einfachen technischen Erwägungen stammt der eingesetzte Sauerstoff im allgemeinen aus der Luft, wobei üblicherweise eine Anreicherung des Sauerstoffs erfolgt. Sie kann beispielsweise durch Luftverflüssigung und anschließender Destillation oder einer Druckwechseladsorption erfolgen. Bevorzugt wird ein Sauerstoff-enthaltendes Gas mit einer Konzentration an Sauerstoff von 20 bis 100 vol.-% verwendet.

Die Belastung des Katalysators mit Kohlenwasserstoffen beträgt im allgemeinen mindstens 20 Nl/l·h, bevorzugt mindestens 30 Nl/l·h, besonders bevorzugt mindestens 35 Nl/l·h.

Der integrale Gesamtumsatz an Kohlenwasserstoffen, d.h. der auf die gesamte Anlage bezogene Umsatz, beträgt beim erfindungsgemäßen Verfahren bei der Variante mit "Rückführung" 80 bis 100%, bevorzugt 90 bis 100%.

Die Abtrennung von Maleinsäureanhydrid kann beispielsweise wie unter (a) beschrieben erfolgen.

Der nach Abtrennung von Maleinsäureanhydrid verbleibende Gasstrom oder wenigstens die darin enthaltenen, nicht-umgesetzten Kohlenwasserstoffe werden bei der Verfahrenvariante mit "Rückführung" mindestens zum Teil zu den Reaktionszonen rückgeführt.

(i) Bei einer Rückführung des Gasstroms ohne Anreicherung der Kohlenwasserstoffe ist es vorteilhaft, einen Teil des Gasstroms aus der Anlage auszuschleusen (sogenannter Purge-Strom), um einer Anreicherung von Verunreinigungen entgegenzusteuern. Der verbleibende Gasstrom kann im allgemeinen zu den Reaktionszonen rückgeführt werden. Die entsprechende Menge an verbrauchtem Kohlenwasserstoff und Sauerstoff wird wie üblich zugefügt.

(ii) Um beispielsweise die Menge an rückzuführendem Inertgas zu verringern ist es gegebenenfalls von Vorteil, die enthaltenen Kohlenwasserstoffe anzureichern. Je nach Art der eingesetzten Kohlenwasserstoffe können verschiedene Methoden in Betracht gezogen werden. Beispielsweise seien genannt das Auskondensieren oder die Adsorption an geeigneten Adsobentien (z.B. auch in Form einer Druckwechsel- oder Temperaturwechseladsorption). So ist etwa zur Anreicherung von n-Butan eine Adsorption an Aktivkohle oder Zeolithe mit anschließender Desorption bei erniedrigtem Druck und/oder erhöhter Temperatur möglich.

Die beiden beschreibenen Verfahrensvarianten "gerader Durchgang" und "Rückführung" stellen zwei bevorzugte Spezialfälle des erfindungsgemäßen Verfahrens dar. Sie wirken keinesfalls limitierend auf andere mögliche Varianten oder auf die als bevorzugt genannten Verfahrensparameter.

Das gewonnene Maleinsäureanhydrid kann weiterverarbeitet werden zu $\gamma$-Butyrolacton, Tetrahydrofuran, 1,4-Butandiol oder Gemischen davon, zum Beispiel durch direkte Hydrierung von Maleinsäureanhydrid in der Gasphase, wie in WO 97/43234 beschrieben oder durch Hydrierung eines Maleinsäurediesters in der Gasphase, wie in WO 97/43242 beschrieben.

In einer besonders bevorzugten Ausführungsform zur Herstellung von Maleinsäureanhydrid setzt man n-Butan als Ausgangs-Kohlenwasserstoff ein und führt die heterogenkatalytische Gasphasenoxidation "geraden Durchgang" an dem erfindungsgemäßen Katalysator durch.

Luft als Sauerstoff- und Inertgas-enthaltendes Gas wird mengengeregelt in die Zufuhr-Einheit gegeben. n-Butan wird ebenfalls mengengeregelt, jedoch in bevorzugt flüssiger Form über eine Pumpe zugeführt und im Gasstrom verdampft. Das Verhältnis zwischen den zugeführten Mengen an n-Butan und Sauerstoff wird im allgemeinen entsprechend der Exothermie der Reaktion und der gewünschten Raum/Zeit-Ausbeute eingestellt und ist daher beispielsweise von der Art und Menge des Katalysators abhängig. Als weitere Komponente wird dem Gasstrom als flüchtige Phosphorverbindung bevorzugt Trialkylphosphat mengengeregelt zugegeben. Die flüchtige Phosphorverbindung kann beispielsweise unverdünnt oder verdünnt in einem geeigneten Lösungsmittel, beispielsweise Wasser, zugegeben werden. Die erforderliche Menge der Phosphor-Verbindung ist von verschiedenen Parametern, beispielsweise der Art und Menge des Katalysators oder den Temperaturen und Drücken in der Anlage, abhängig und für jedes System zu adaptieren.

Der Gasstrom wird zur innigen Durchmischung durch einen statischen Mischer und zur Aufheizung durch einen Wärmetauscher geleitet. Der durchmischte und vorgeheizte Gasstrom wird nun zum Rohrbündelreaktor geleitet, in dem sich der erfindungsgemäße Katalysator befindet. Der Rohrbündelreaktor wird durch einen Salzschmelzen-Kreislauf temperiert. Die Temperatur wird derart eingestellt, daß bevorzugt ein Umsatz pro Reaktordurchgang von 75 bis 90% erreicht wird.

Der aus dem Rohrbündelreaktor stammende Produktgasstrom wird in einem Wärmetauscher heruntergekühlt und der Einheit zur Abtrennung des Maleinsäureanhydrids zugeführt. Die Einheit enthält in der bevorzugten Ausführungsform mindestens einen Apparat zur absorptiven Entfernung des Maleinsäureanhydrids und gegebenenfalls der oxygenierten Kohlenwasserstoff-Nebenprodukte. Geeignete Apparate sind beispielsweise mit einer Absorptionsflüssigkeit gefüllte Behälter, durch die das heruntergekühlte Austragsgas geleitet wird oder Apparate, in denen die Absorptionsflüssigkeit in den Gasstrom eingesprüht wird. Die Maleinsäureanhydrid-haltige Lösung wird zur weiteren Verarbeitung oder zur Isolierung des Wertprodukts aus der Anlage ausgeschleust. Der verbleibende Gasstrom wird ebenfalls aus der Anlage ausgeschleust und gegebenenfalls einer Einheit zur Rückgewinnung des nicht-umgesetzten n-Butans zugeführt.

Das erfindungsgemäße Verfahren unter Verwendung der erfindungsgemäßen Katalysatoren ermöglicht eine hohe Kohlenwasserstoff-Belastung des Katalysators bei einem hohen Umsatz, einer hohen Selektivität, einer hohen Ausbeute und daher auch einer hohen Raum/Zeit-Ausbeute an Maleinsäureanhydrid.

Definitionen

Die in dieser Schrift verwendeten Größen sind, falls nicht anders erwähnt, wie folgt definiert:

$$\text{geometrische Oberfläche } A_{geo} = \frac{d_1^2 - d_2^2}{2}\pi + (d_1 + d_2)\pi h$$

$$\text{geometrisches Volumen } V_{geo} = \frac{d_1^2 - d_2^2}{4}\pi h$$

$$\text{theoretisches Volumen Vollzylinder } V_{overall} = \frac{d_1^2}{4}\pi h$$

$$\text{Raum/Zeit-Ausbeute} = \frac{m_{Maleinsäureanhydrid}}{V_{Katalysator} \cdot t}$$

$$\text{Belastung} = \frac{V_{Kohlenwasserstoff}}{V_{Katalysator} \cdot t}$$

$$\text{Umasatz } U = \frac{n_{KW,\ Reaktor,\ ein} - n_{KW,\ Reaktor,\ aus}}{n_{KW,\ Reaktor,\ ein}}$$

$$\text{Selektivität } S = \frac{n_{MSA,Reaktor,\ aus}}{n_{KW,\ Reaktor,\ ein} - n_{KW,\ Reactor,\ aus}}$$

$$\text{Ausbeute } A = U \cdot S$$

| | |
|---|---|
| $d_1$ | äußerer Durchmesser des Hohlzylinders bzw. Vollzylinders [mm] |
| h | Höhe des Hohlzylinders bzw. Vollzylinders [mm] |
| $d_2$ | Durchmesser der hindurchgehenden Öffnung [mm] |
| $A_{geo}$ | geometrische Oberfläche der Formkörper unter Zugrundelegung der geometrischen Größen $d_1$, h und $d_2$ [$mm^2$] |
| $V_{geo}$ | geometrisches Volumen der Formkörper unter Zugrundelegung der geometrischen Größen $d_1$, h und $d_2$ [$mm^3$] |
| $V_{overall}$ | theoretisches Volumen eines entsprechenden Vollzylinders mit Höhe h und äußerem Durchmesser $d_1$ [$mm^3$] |
| $m_{Maleinsäureanhydrid}$ | Masse an produziertem Maleinsäureanhydrid [g] |
| $V_{Katalysator}$ | Schüttvolumen Katalysator, summiert über alle Reaktionszonen [1] |
| t | Zeiteinheit [h] |
| $V_{Kohlenwasserstoff}$ | auf 0°C und 0,1013 MPa normiertes Volumen des Kohlenwasserstoffs in der Gasphase [N1] (Rechnerische Größe. Liegt ein Kohlenwasserstoff unter diesen Bedingungen in der Flüssigphase vor, so wird über das ideale Gasgesetz das hypothetische Gasvolumen berechnet.) |
| U | Umsatz an Kohlenwasserstoffen pro Reaktordurchgang |

| | |
|---|---|
| S | Selektivität bzgl. Maleinsäureanhydrid pro Reaktordurchgang |
| A | Ausbeute an Maleinsäureanhydrid pro Reaktordurchgang |
| $n_{KW, Reaktor, ein}$ | Stoffmengenstrom an Kohlenwasserstoffen am Reaktoreingang [mol/h] |
| $n_{KW, Reaktor, aus}$ | Stoffmengenstrom an Kohlenwasserstoffen am Reaktorausgang [mol/h] |
| $n_{KW, Anlage, ein}$ | Stoffmengenstrom an Kohlenwasserstoffen am Eingang der Anlage [mol/h] |
| $n_{KW, Anlage, aus}$ | Stoffmengenstrom an Kohlenwasserstoffen am Ausgang der Anlage [mol/h] |
| $n_{MSA, Reaktor, aus}$ | Stoffmengenstrom an Maleinsäureanhydrid am Reaktorausgang [mol/h] |
| $n_{MSA, Anlage, aus}$ | Stoffmengenstrom an Maleinsäureanhydrid am Ausgang der Anlage [mol/h] |

Beispiele

Katalysatoren A bis F

In einem 240 l Kessel wurden unter Rühren 11,8 kg 100%ige Orthophosphorsäure in 150 l Isobutanol gelöst und anschließend 9,09 kg Vanadiumpentoxid zugegeben. Diese Suspension wurde 16 Stunden unter Rückfluß erhitzt und dann auf Raumtemperatur abgekühlt. Der entstandenen Niederschlag wurde abfiltriert, mit Isobutanol gewaschen und bei 150°C im Vakuum bei 8 kPa (80 mbar) getrocknet. Anschließend wurde das getrocknete Pulver 2 Stunden bei 250 bis 300°C in einem Drehrohr behandelt. Nach dem Abkühlen auf Raumtemperatur wurden 3 Gew.-% Graphit zugegeben und innig vermischt.

Das Pulver wurde anschließend zu Hohlzylindern mit unterschiedlicher Geometrie tablettiert.

Die Hohlzylinder der verschiedenen Geometrien wurden nach der Formgebung in einem Muffelofen zunächst unter Luft mit 7°C/min auf 250°C und anschließend mit 2°C/min auf 350°C erhitzt. Bei dieser Temperatur wurde der Katalysator für 10 Minuten belassen, bevor die Atmosphäre von Luft auf $N_2/H_2O$ (1:1) umgestellt wurde. Unter der $N_2/H_2O$-Atmosphäre (1:1) wurde auf 425°C erhitzt und das System für 3 Stunden bei dieser Temperatur belassen. Zum Schluß wurde unter Stickstoff auf Raumtemperatur abgekühlt.

Tabelle 1 zeigt eine Übersicht der geometrischen und physikalisch-chemischen Eigenschaften der hergestellten Katalysatoren.

Anlage

Die Versuchsanlage war ausgestattet mit einer Zufuhr-Einheit und einem Reaktorrohr. Der Ersatz eines Rohrbündelreaktors durch ein Reaktorrohr ist im Labor- oder Technikumsmaßstab sehr gut möglich, sofern die Abmessungen des Reaktorrohres im Bereich eines technischen Reaktorrohres liegen. Die Anlage wurde im "geraden Durchgang" betrieben.

Der Kohlenwasserstoff wurde mengengeregelt in flüssiger Form über eine Pumpe zugegeben. Als Sauerstoffhaltiges Gas wurde Luft mengengeregelt zugegeben. Triethylphosphat (TEP) wurde ebenfalls mengengeregelt, gelöst in Wasser, in flüssiger Form zugegeben.

Die Rohrbündelreaktor-Einheit bestand aus einem Rohrbündelreaktor mit einem Reaktorrohr. Die Länge des Reaktorrohrs betrug 6,5 m, der Innendurchmesser 22,3 mm. Innerhalb des Reaktorrohres befand sich in einem Schutzrohr ein Multi-Thermoelement mit 20 Temperaturmeßstellen. Das Reaktorrohr war von einem temperierbaren Wärmeträger-Kreislauf umgeben und wurde von dem Reaktionsgasgemisch von oben nach unten durchströmt. Die oberen 0,3 m des Reaktorrohres waren mit Inertmaterial gefüllt und bildeten die Vorheizzone. Die Reaktionszone enthielt 2,2 l Katalysator. Als Wärmeträgermedium wurde eine Salzschmelze eingesetzt.

Direkt nach der Rohrbündelreaktor-Einheit wurde gasförmiges Produkt entnommen und der gaschromatographischen on-line Analytik zugeführt. Der Hauptstrom des gasförmigen Reaktoraustrags wurde aus der Anlage ausgeschleust.

Beispiele 1 bis 6

Alle Beispiele wurden mit n-Butan als Kohlenwasserstoff durchgeführt. Die erhaltenen Ergebnisse sind in Tabelle 2 zusammengefaßt.

Zur Vergleichbarkeit der Versuche wurde über die Salzbadtemperatur $T_{SB}$ ein Umsatz von etwa 85% eingestellt. Die Versuche zeigen, daß die Vergleichskatalysatoren E* und F*, welche durch ein $A_{geo}/V_{geo}$-Verhältnis von kleiner 2 $mm^{-1}$ (Vergleichskatalysator E*) beziehungsweise ein $h/d_2$-Verhältnis von größer 1,5 (Vergleichskatalysator F*) charakterisiert sind, zu einer deutlich niedrigeren Selektivität, Ausbeute und Raum/Zeit-Ausbeute führen als die erfindungsgemäßen Katalysatoren A bis D.

Tabelle 1: Übersicht der geometrischen und physikalisch-chemischen Eigenschaften der hergestellten Katalysatoren

| Beispiel / Katalysator | $d_1$ [mm] | h [mm] | $d_2$ [mm] | $A_{geo}/V_{geo}$ [$mm^{-1}$] | $h/d_2$ | $V_{geo}/V_{overal}$ I | $V_{ox.}$ | BET [$m^2/g$] | Dichte [kg/l] |
|---|---|---|---|---|---|---|---|---|---|
| 1 / A | 5 | 3 | 2 | 2,00 | 1,5 | 0,84 | 4,21 | 23 | 0,70 |
| 2 / B | 5 | 3 | 2,5 | 2,27 | 1,2 | 0,75 | 4,10 | 23 | 0,65 |
| 3 / C | 5 | 3 | 3 | 2,67 | 1,0 | 0,64 | 4,28 | 20 | 0,59 |
| 4 / D | 6 | 3 | 3 | 2,00 | 1,0 | 0,75 | 4,21 | n.b. | 0,68 |
| 5* / E* | 8 | 5 | 5 | 1,73* | 1,0 | 0,61 | 4,13 | 31 | 0,52 |
| 6* / F* | 4 | 4 | 1,2 | 2,18 | 3,3* | 0,84 | 4,15 | 21 | 0,80 |

* Vergleichsbeispiel / Vergleichskatalysator    n.b.: nicht bestimmt.

Es wurden folgende Abkürzungen verwendet:

$V_{ox.}$ = mittlere Oxidationsstufe des Vanadiums

BET = BET-Oberfläche

Dichte = Schüttdichte

Tabelle 2: Übersicht der geometrischen und katalytischen Eigenschaften der getesteten Katalysatoren

| Beispiel / Katalysator | $d_1$ [mm] | h [mm] | $d_2$ [mm] | $T_{SB}$ [°C] | $T_{HP}$ [°C] | Δp [MPa] | Umsatz U [%] | Selektivität S [%] | Ausbeute A [%] | Raum/Zeit-Ausbeute [g/l·h] |
|---|---|---|---|---|---|---|---|---|---|---|
| 1 / A | 5 | 3 | 2 | 410 | 434 | 0,15 | 84,9 | 65,0 | 55,2 | 97 |
| 2 / B | 5 | 3 | 2,5 | 410 | 434 | 0,13 | 85,1 | 67,3 | 57,3 | 100 |
| 3 / C | 5 | 3 | 3 | 400 | 429 | 0,12 | 85,0 | 63,4 | 53,9 | 94 |
| 4 / D | 6 | 3 | 3 | 400 | 429 | 0,11 | 85,0 | 64,8 | 55,1 | 96 |
| 5* / E* | 8 | 5 | 5 | 420 | n.b. | 0,07 | 85,4 | 55,0 | 47,0 | 82 |
| 6* / F* | 4 | 4 | 1,2 | 405 | 440 | 0,16 | 85,1 | 60,2 | 51,2 | 90 |

* Vergleichsbeispiel / Vergleichskatalysator n.b.: nicht bestimmt.

Es wurden folgende Abkürzungen verwendet:

$T_{SB}$ = Temperatur des Salzbades
$T_{HP}$ = Temperatur des Heißpunktes (Hotspot)
Δp = Druckverlust im Reaktor

Die Versuche wurden unter folgenden Bedingungen durchgeführt: Konzentration an n-Butan = 2,0 Vol.-%
GHSV = 2000 NL/$l_{Katalysator}$·h
Druck = 0,2 MPa abs
Konzentration an Triethylphosphat (TEP) = 2 Volumen-ppm

## Patentansprüche

1. Katalysator für die Herstellung von Maleinsäureanhydrid durch heterogenkatalytische Gasphasenoxidation eines Kohlenwasserstoffs mit mindestens vier Kohlenstoffatomen, der eine katalytisch aktive Masse enthaltend Vanadium, Phosphor und Sauerstoff umfasst und eine im wesentlichen hohlzylinderförmige Struktur aufweist, **dadurch gekennzeichnet, daß** die hohlzylinderförmige Struktur (a) ein Verhältnis der Höhe h zum Durchmesser der hindurchgehenden Öffnung $d_2$ von höchstens 1,5 und (b) ein Verhältnis der geometrischen Oberfläche $A_{geo}$ zum geometrischen Volumen $V_{geo}$ von mindestens 2 $mm^{-1}$ aufweist.

2. Katalysator nach Anspruch 1, **dadurch gekennzeichnet, daß** das Verhältnis des geometrischen Volumens $V_{geo}$ der hohlzylinderförmigen Struktur zum theoretischen Volumen $V_{overall}$ eines entsprechenden Vollzylinders mit gleicher Höhe h und gleichem äußeren Durchmesser $d_1$ höchstens 0,85 beträgt.

3. Katalysator nach den Ansprüchen 1 bis 2, **dadurch gekennzeichnet, daß** der äußere Durchmesser $d_1$ 3 bis 10 mm, die Höhe h 1 bis 10 mm und der Durchmesser der inneren Öffnung $d_2$ 1 bis 8 mm beträgt.

4. Katalysator nach den Ansprüchen 1 bis 3, **dadurch gekennzeichnet, daß** das Phosphor/Vanadium-Atomverhältnis 0,9 bis 1,5, die mittlere Oxidationsstufe des Vanadiums +3,9 bis +4,4, die BET-Oberfläche 10 bis 50 $m^2$/g, das Porenvolumen 0,1 bis 0,5 ml/g und die Schüttdichte 0,5 bis 1,5 kg/l beträgt.

5. Verfahren zur Herstellung von Maleinsäureanhydrid durch heterogenkatalytische Gasphasenoxidation eines Kohlenwasserstoffs mit mindestens vier Kohlenstoffatomen mit Sauerstoff enthaltenden Gasen, **dadurch gekennzeichnet, daß** man einen Katalysator gemäß den Ansprüchen 1 bis 4 einsetzt.

6. Verfahren nach Anspruch 5, **dadurch gekennzeichnet, daß** man die heterogenkatalytische Gasphasenoxidation in einem Rohrbündelreaktor bei einer Temperatur von 350 bis 480°C und einem Druck von 0,1 bis 1,0 MPa abs durchführt.

7. Verfahren nach den Ansprüchen 5 bis 6, **dadurch gekennzeichnet, daß** man als Kohlenwasserstoff n-Butan einsetzt.

8. Verfahren nach den Ansprüchen 5 bis 7, **dadurch gekennzeichnet, daß** man die heterogenkatalytische Gasphasenoxidation in Gegenwart einer flüchtigen Phosphorverbindung durchführt.

## Claims

1. A catalyst for preparing maleic anhydride by heterogeneously catalyzed gas-phase oxidation of a hydrocarbon having at least four carbon atoms, which comprises a catalytically active composition comprising vanadium, phosphorus and oxygen and has an essentially hollow cylindrical structure, wherein the hollow cylindrical structure has (a) a ratio of the height h to the diameter of the continuous hole $d_2$ of not more than 1.5 and (b) a ratio of the geometric surface area $A_{geo}$ to the geometric volume $V_{geo}$ of at least 2 $mm^{-1}$.

2. A catalyst as claimed in claim 1, wherein the ratio of the geometric volume $V_{geo}$ of the hollow cylindrical structure to the theoretical volume $V_{overall}$ of a corresponding solid cylinder having the same height h and the same external diameter $d_1$ is not more than 0.85.

3. A catalyst as claimed in claim 1 or 2, wherein the external diameter $d_1$ is from 3 to 10 mm, the height h is from 1 to 10 mm and the diameter of the internal hole $d_2$ is from 1 to 8 mm.

4. A catalyst as claimed in any of claims 1 to 3, wherein the phosphorus/vanadium atomic ratio is from 0.9 to 1.5, the mean oxidation state of the vanadium is from +3.9 to +4.4, the BET surface area is from 10 to 50 $m^2$/g, the pore volume is from 0.1 to 0.5 ml/g and the bulk density is from 0.5 to 1.5 kg/l.

5. A process for preparing maleic anhydride by heterogeneously catalyzed gas-phase oxidation of a hydrocarbon having at least four carbon atoms by means of oxygen-containing gases, wherein a catalyst as claimed in any of claims 1 to 4 is used.

6. A process as claimed in claim 5, wherein the heterogeneously catalyzed gas-phase oxidation is carried out in a shell-and-tube reactor at from 350 to 480°C and a pressure of from 0.1 to 1.0 MPa abs.

7. A process as claimed in claim 5 or 6, wherein the hydrocarbon used is n-butane.

8. A process as claimed in any of claims 5 to 7, wherein the heterogeneously catalyzed gas-phase oxidation is carried out in the presence of a volatile phosphorus compound.

## Revendications

1. Catalyseur pour la préparation d'anhydride maléique au moyen d'une oxydation en phase gazeuse par catalyse hétérogène d'un hydrocarbure possédant au moins quatre atomes de carbone, qui présente une masse à activité catalytique contenant du vanadium, du phosphore et de l'oxygène et qui présente essentiellement une structure de cylindre creux, **caractérisé en ce que** la structure de cylindre creux présente (a) un rapport de la hauteur h au diamètre de l'ouverture transversale $d_2$ d'au maximum 1,5 et (b) un rapport de la surface géométrique $A_{geo}$ au volume géométrique $V_{geo}$ d'au moins 2 $mm^{-1}$.

2. Catalyseur selon la revendication 1, **caractérisé en ce que** le rapport du volume géométrique $V_{geo}$ de la structure de cylindre creux au volume théorique $V_{overall}$ d'un cylindre plein correspondant, présentant la même hauteur h et le même diamètre externe $d_1$, est d'au maximum 0,85.

3. Catalyseur selon les revendications 1 à 2, **caractérisé en ce que** le diamètre externe $d_1$ va de 3 mm à 10 mm, la hauteur h va de 1 mm à 10 mm et le diamètre de l'ouverture interne $d_2$ va de 1 mm à 8 mm.

4. Catalyseur selon les revendications 1 à 3, **caractérisé en ce que** le rapport atomique phosphore/vanadium va de 0,9 à 1,5, le degré d'oxydation moyen du vanadium va de + 3,9 à + 4,4, la surface spécifique BET va de 10 $m^2$/g à 50 $m^2$/g, le volume des pores va de 0,1 mL/g à 0,5 mL/g et la masse volumique apparente va de 0,5 kg/L à 1,5 kg/L.

5. Procédé de préparation de l'anhydride maléique au moyen d'une oxydation en phase gazeuse par catalyse hétérogène d'un hydrocarbure possédant au moins quatre atomes de carbone avec des gaz contenant de l'oxygène, **caractérisé en ce que** l'on met en oeuvre un catalyseur selon les revendications 1 à 4.

6. Procédé selon la revendication 5, **caractérisé en ce que** l'on réalise l'oxydation en phase gazeuse par catalyse hétérogène dans un réacteur à faisceau de tubes à une température allant de 350°C à 480°C et sous une pression allant de 0,1 MPa à 1,0 MPa.

7. Procédé selon les revendications 5 à 6, **caractérisé en ce que** l'on met en oeuvre le n-butane en tant qu'hydrocarbure.

8. Procédé selon les revendications 5 à 7, **caractérisé en ce que** l'on réalise l'oxydation en phase gazeuse par catalyse hétérogène en présence d'un composé volatil du phosphore.